(19) Europäisches Patentamt
European Patent Office
Office européen des brevets

(11) Veröffentlichungsnummer: **0 009 168**
**A1**

# (12) EUROPÄISCHE PATENTANMELDUNG

(21) Anmeldenummer: **79103279.0**

(22) Anmeldetag: **05.09.79**

(51) Int. Cl.³: **C 07 C 93/06**, A 61 K 31/13

(30) Priorität: **11.09.78 DE 2839475**

(43) Veröffentlichungstag der Anmeldung: **02.04.80 Patentblatt 80/7**

(84) Benannte Vertragsstaaten: **AT BE CH FR GB IT LU NL**

(71) Anmelder: **DOLORGIET ARZNEIMITTELFABRIK PETER DOLL KG, Postfach 20 07 44, D-5300 Bonn 2 Bad Godesberg (DE)**

(72) Erfinder: **Kunz, Wilhelm, Dr., Waldstrasse 17, D-5307 Wachtberg-Villiprot (DE)**
Erfinder: **Gruber, Klaus, Dr., Dipl.-Chem., Am Botten 10, D-5300 Bonn 2 (DE)**

(74) Vertreter: **Kraus, Walter, Dr. et al, Patentanwälte Dres. Kraus & Weisert Irmgardstrasse 15, D-8000 München 71 (DE)**

## (54) Isopropylamin-Verbindungen, Verfahren zu ihrer Herstellung und sie enthaltende Arzneimittel.

(57) Die Erfindung betrifft neue Isopropylamin-Verbindungen der allgemeinen Formel I

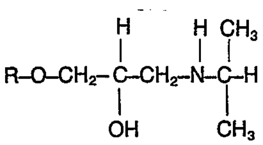

I

worin R ein Nitrophenylrest ist, der ein oder mehrfach durch Halogene, vorzugsweise Chlor, und/oder niedere Alkylreste mit 1 bis 4 C-Atomen, gerade oder verzweigt, und/oder Alkoxyreste mit 1 bis 3 C-Atomen, substituiert sein kann, sowie deren Salze mit physiologisch indifferenten Säuren. Die Erfindung betrifft weiterhin Verfahren zur Herstellung der neuen Verbindungen und sie enthaltende pharmazeutische Präparate.

EP 0 009 168 A1

0009168

Isopropylamin-Verbindungen, Verfahren zu ihrer Herstellung und sie enthaltende Arzneimittel

Die Erfindung betrifft neue Isopropylamin-Verbindungen der allgemeinen Formel I, Verfahren zu ihrer Herstellung und ihre pharmazeutische Anwendung,

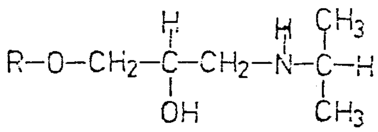

I

worin R ein Nitrophenylrest ist, der ein oder mehrfach durch Halogene, vorzugsweise Chlor, und / oder niedere Alkylreste mit 1 bis 4 C-Atomen, gerade oder verzweigt, und / oder Alkoxyreste mit 1 bis 3 C-Atomen, substituiert sein kann, sowie deren Salze mit physiologisch indifferenten Säuren.

Die erfindungsgemäßen Produkte der Formel I können nach folgenden an sich bekannten Verfahren erhalten werden, wobei R in den nachfolgenden Formeln die gleiche Bedeutung hat wie in Formel I.

1) Durch Umsetzung von Isopropylamin mit einem Arylglycidaether der allgemeinen Formel II

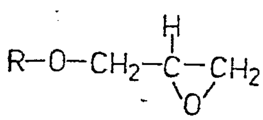

II

bei erhöhter Temperatur, gegebenenfalls in Anwesenheit eines inerten Lösungsmittels, oder mit einer Verbindung der allgemeinen Formel III

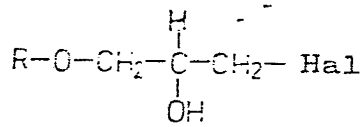

III

worin Hal für Chlor, Brom oder Jod steht,

bei erhöhter Temperatur, gegebenenfalls in Anwesenheit eines inerten Lösungsmittels und / oder eines Säurefängers.

2) Durch Umsetzung des Isopropylamino-2.3-oxydopropan oder des Isopropylamino-2-hydroxy-propan-halogenid-(3) mit einem Phenol der allgemeinen Formel

R-OH IV

bei erhöhter Temperatur, gegebenenfalls in Anwesenheit eines inerten Lösungsmittels und / oder in einem alkalischen Milieu.

3) Durch Umsetzung einer Verbindung der allgemeinen Formel

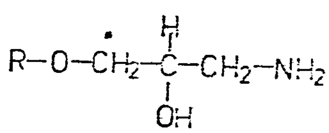

V

mit einem Isopropyl-halogenid, vorzugsweise dem Chlorid, bei erhöhter Temperatur, gegebenenfalls in Anwesenheit eines inerten Lösungsmittels und / oder eines Säurefängers.

4) Durch Reduktion einer Verbindung der allgemeinen Formel VI

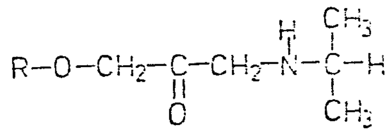

VI

zum entsprechenden Alkohol durch z.B. Hydrierung in Anwesenheit eines geeigneten Katalysators, wie z.B. Platinoxyd, gegebenenfalls in Gegenwart eines inerten Lösungsmittels.

5) Durch Hydrolyse von Oxazolidonen der allgemeinen Formel VII.

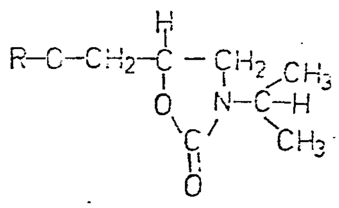

VII

Aus den Basen können Salze mit physiologisch verträglichen anorganischen oder organischen Säuren hergestellt werden.

Als Beispiele für physiologisch unbedenkliche anorganische und organische Säuren, die zur Salzbildung geeignet sind, seien genannt, Chlorwasserstoff-, Bromwasserstoff-, Jodwasserstoff-, Schwefel-, Salpeter-, Phosphor-, Essig-, Glukon-, Milch-, Malon-, Malein-, Bernstein-, Fumar-, Wein-, Zitronen-, Benzoe-, ß-Oxy-naphthol-, Embon- oder Theophyllinessigsäure.

Die erfindungsgemäßen Verbindungen treten entsprechend dem Asymmetriezentrum am Kohlenstoffatom 2 der Seitenkette in optisch aktiven Isomeren auf, die nach bekannten Verfahren aufgetrennt werden können.

Die erfindungsgemäßen Isopropylamin-Derivate und ihre physiologisch unbedenklichen Salze weisen eine hohe ß-adrenergische Blockierungsaktivität auf und haben die Fähigkeit, die Blutplättchenaggregation zu hemmen bzw. zu verhindern.

Beispiel:

10,5 g 1-(2-Chlor-5-nitro-phenoxy)-2.3-epoxypropan werden mit 5 g Isopropylamin in 100 ml Aethanol ca. 8 Stunden am Rückfluß erhitzt. Nach Abdestillieren des Alkohols im Vakuum wird der Rückstand in Benzol gelöst. In diese Benzol-Lösung leitet man unter Eiskühlung Chlorwasserstoff bis zur Sättigung ein. Der Niederschlag wird abgesaugt, mit Aceton gewaschen und aus Isopropanol umkristallisiert.

FP. 163 - 164° C

Analyse: $C_{13}H_{20}N_2O_4$ X HCl Mol.-Gew. 304,781

| | ber. | gef. |
|---|---|---|
| C | 51,23 % | 51,28 % |
| H | 6,95 % | 7,03 % |
| N | 9,19 % | 9,09 % |

Patentansprüche :

1) Neue Isopropylamin-Derivate der allgemeinen Formel I

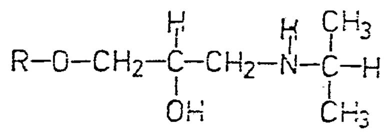

I

worin R ein Nitrophenylrest ist, der ein oder mehrfach durch Halogen, vorzugsweise Chlor, und / oder niedere Alkylreste mit 1 bis 4 C-Atomen, gerade oder verzweigt, und / oder Alkoxyreste mit 1 bis 3 C-Atomen, substituiert sein kann, sowie deren Salze mit physiologisch indifferenten Säuren.

2) Verfahren zur Herstellung der Verbindungen der allgemeinen Formel I durch Umsetzung von Isopropylamin mit einem Arylglycidaether der allgemeinen Formel II

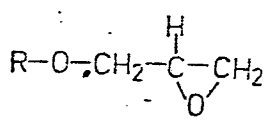

II

bei erhöhter Temperatur, gegebenenfalls in Anwesenheit eines inerten Lösungsmittels oder mit einer Verbindung der allgemeinen Formel III,

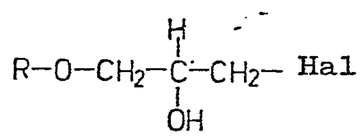

III

worin Hal für Chlor, Brom oder Jod steht,

bei erhöhter Temperatur, gegebenenfalls in Anwesenheit eines inerten Lösungsmittels und / oder eines Säurefängers, wobei R die gleiche Bedeutung hat wie in der allgemeinen Formel I.

3) Verfahren zur Herstellung der Verbindungen der allgemeinen Formel I durch Umsetzung des Isopropylamino-2.3-oxydopropan oder des Isopropylamino-2-hydroxy-propan-halogenid-(3) mit einem Phenol der allgemeinen Formel

$$R{-}OH \qquad IV$$

bei erhöhter Temperatur, gegebenenfalls in Anwesenheit eines inerten Lösungsmittels und / oder in einem alkalischen Milieu, wobei R die gleiche Bedeutung hat wie in der allgemeinen Formel I.

4) Verfahren zur Herstellung von Verbindungen der allgemeinen Formel I durch Umsetzung einer Verbindung der allgemeinen Formel V

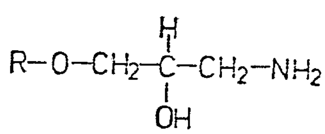

V

mit einem Isopropyl-halogenid, vorzugsweise dem Chlorid, bei erhöhter Temperatur, gegebenenfalls in Anwesenheit eines inerten Lösungsmittels und / oder eines Säurefängers, wobei R der Bedeutung der allgemeinen Formel I entspricht.

5) Verfahren zur Herstellung von Verbindungen der allgemeinen Formel I durch Reduktion einer Verbindung der allgemeinen Formel VI

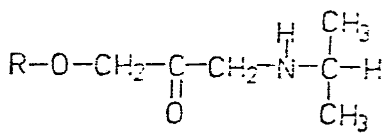

VI

zum entsprechenden Alkohol durch z.B. Hydrierung in Anwesenheit eines geeigneten Katalysators, wie z.B. Platinoxyd, gegebenenfalls in Gegenwart eines inerten Lösungsmittels. R ist gleich der allgemeinen Formel I.

6) Verfahren zur Herstellung von Verbindungen der allgemeinen Formel I durch Hydrolyse von Oxazolidonen der allgemeinen Formel VII

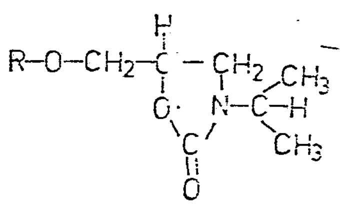

VII .

R hat die in der allgemeinen Formel I angegebene Bedeutung.

7) Salze mit physiologisch verträglichen anorganischen und organischen Säuren.

8) Optisch aktive Verbindungen, die infolge des Asymmetriezentrums am Kohlenstoffatom 2 der Seitenkette auftreten.

9) Pharmazeutische Präparate aus diesen Verbindungen.

0009168

Europäisches Patentamt

**EUROPÄISCHER RECHERCHENBERICHT**

Nummer der Anmeldung

EP 79 10 3279

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| X | DE - A - 1 543 357 (BOEHRINGER)<br>* Seiten 1-7; Beispiele 3,6,12, 20 *<br>-- | 1,4,6-9 |
| X | DE - A - 1 593 762 (BOEHRINGER)<br>* Seiten 1-9; Beispiele 8,22, 23 *<br>-- | 1-4,6-9 |
| X | FR - M - 3647 (BOEHRINGER)<br>* Seiten 1,2; Beispiel 5; Seite 5, Nr. 4,15 *<br>-- | 1-9 |
| X | CHEMICAL ABSTRACTS, Vol. 74, Nr. 11, 15. März 1971, Seite 195, Nr. 51825h<br>Columbus, Ohio, U.S.A.<br>A.M. BARRETT et al.: "Effect of ring substitution in 1-isopropylamino-3-phenoxypropan-2-ol on β-adrenoceptor inhibition"<br>& EUR. J. PHARMACOL. 1970, 12(3), 372-4 (Eng.)<br>* Zusammenfassung *<br>-- | 1,9 |
| X | CHEMICAL ABSTRACTS, Vol. 77, Nr. 23, 4. Dezember 1972, Seite 2, Nr. 147390a<br>Columbus, Ohio, U.S.A.<br>R. GRYGLEWSKI: "Pharmacological studies of the adrenergic receptor structure in the heart"<br>& ACTA PHYSIOL. POL., SUPL. 1972, 23(4), 23-42<br>* Zusammenfassung *<br>---- | 1,9 |

**KLASSIFIKATION DER ANMELDUNG (Int.Cl. 3)**

C 07 C 93/06
A 61 K 31/13

**RECHERCHIERTE SACHGEBIETE (Int. Cl. 3)**

C 07 C 93/06
A 61 K 31/13

**KATEGORIE DER GENANNTEN DOKUMENTE**

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

ⓧ Der vorliegende Recherchenbericht wurde für alle Patentansprüche erstellt.

| Recherchenort | Abschlußdatum der Recherche | Prüfer |
|---|---|---|
| Den Haag | 03-12-1979 | MOREAU |